(19) Europäisches Patentamt

European Patent Office

Office européen des brevets

(11) Publication number: **0 481 600 A2**

## EUROPEAN PATENT APPLICATION

(21) Application number: **91308345.7**

(22) Date of filing: **12.09.91**

(51) Int. Cl.5: **A61L 15/28**, A61L 15/26, A61L 15/44, A61K 47/36, A61K 47/34

(30) Priority: **16.10.90 GB 9022368**

(43) Date of publication of application:
**22.04.92 Bulletin 92/17**

(84) Designated Contracting States:
**AT BE CH DE DK ES FR GB GR IT LI LU NL SE**

(71) Applicant: **AMERICAN CYANAMID COMPANY
Berdan Avenue
Wayne New Jersey 06904(US)**

(72) Inventor: **Payne, Nicholas Ian
"Brackley" 215 Swanwick Lane Lower
Swanwick
Southampton Hampshire S03 7DP(GB)**
Inventor: **Gibson, Mark
19 Smith Way Grange Farm Shepshed
Loughborough, LE12 9TO(GB)**
Inventor: **Taylor, Peter Mark
7 Haven Park Hillcrest
East Kilbride G75 8PO(GB)**

(74) Representative: **Allam, Peter Clerk et al
LLOYD WISE, TREGEAR & CO. Norman
House 105-109 Strand
London WC2R 0AE(GB)**

(54) **Materials useful in human and veterinary medicine.**

(57) There is provided a solid material useful in medicine, especially a film material for use as a wound dressing or as a drug delivery vehicle, which comprises a molecular mixture of (a) a compound which has film-forming properties, for example hydroxyethylcellulose, hydroxypropylmethylcellulose or polyvinyl alcohol, and (b) a polyoxyethylene-polyoxypropylene block copolymer or a tetrasubstituted derivative of ethylene-diamine in which the substituents are block copolymers of polyoxyethylene-polyoxypropylene, as a modifier. The molecular mixture may be formed, for example, by evaporating a solution of the two components (a) and (b) in a solvent, or by cooling a liquid composition comprising at least one of components (a) and (b) in molten form from an elevated temperature.

This invention relates to a new material which is useful in a number of medical and veterinary applications, for instance as a primary or secondary wound dressing, as a surgical drape, or as a drug delivery device.

Recent years have seen the development of wound dressings which consist of a transparent, semi-permeable film of polyurethane or other synthetic polymer, often coated on one side with an adhesive by which the dressing can be adhered to the wound site. These film dressings are now widely used in the treatment of wounds and burns, their permeability to air and water vapour allowing the tissue to remain in an environment conducive to optimum wound healing. However, the known film dressings can be difficult to apply, particularly to certain areas such as around fingers, toes and joints, because they have a tendency to adhere to themselves. Furthermore, they may allow infections to develop beneath the film as a result of tracking of bacteria along ridges and folds in the dressing if good adhesion to the wound has not been achieved.

Another recently introduced type of wound dressing are the so-called gel dressings. These are available either as gels per se or as self-supporting sheet-like products. Although possessing some advantages, many of the presently available gel dressings, even those provided as self-supporting sheets, have the disadvantage of lacking mechanical strength, and consequently, and in contrast to the film dressings discussed above, they often require an additional dressing to hold them in place. Further, these gel dressings can be difficult to apply and in some instances leave residues which may become implanted in new tissue growth.

The currently available film and gel wound dressings have been described in more detail in the literature, see for example Lawrence in "Pharmacy Update", April 1987, pages 147-150; and Turner in "Pharmacy International", June 1985, pages 131-134.

A surgical drape is a sterile material which is laid over the area surrounding a wound or incision during surgery in order to decrease the risk of microbial contamination. Such surgical drapes are conventionally formed either of woven materials or of hydrophobic polymer films, but neither type of material is wholly satisfactory. Surgical drapes formed from woven materials suffer from a number of disadvantages, e.g. they need to be re-sterilized after use, they are difficult to conform to wound contours and they may leave behind fabric residues. Surgical drapes in the form of hydrophobic polymer films may be difficult to apply, when applied they can form ridges along which bacteria can track, and when finished with they can be difficult to remove without causing trauma because of their adhesive bonding properties.

The present invention provides a novel material which can be provided in film or sheet form for use as a wound dressing or as a surgical drape, or as a drug delivery system, or which alternatively may be provided in various different forms, e.g. as cast blocks or extruded or moulded shapes which also find utility in human or veterinary medicine, as will become apparent hereinafter.

More particularly, the present invention provides a solid material which comprises a molecular mixture of (a) one or more compounds having film-forming properties, with (b) one or more modifying compounds selected from (i) block copolymers of polyoxyethylene-polyoxypropylene and (ii) tetrasubstituted derivatives of ethylene diamine wherein the substituents are block copolymers of polyoxyethylene-polyoxypropylene, the weight ratio of the component (a) to the component (b) in the mixture being from 1:20 to 25:1.

As already indicated, the materials of the present invention may be provided in a variety of different forms, using a number of different manufacturing techniques. Generally, in accordance with this invention, molecules of the compound or compounds constituting the film-forming component are intimately admixed with molecules of the compound or compounds constituting the modifying component. Such molecular mixing of the two essential components can be readily achieved by first forming a liquid mixture of the two components, and then causing the mixture to solidify.

For instance, solid material in accordance with this invention can be formed by heating either the film-forming component or the modifying component to an elevated temperature at which it is molten, but not degraded, thoroughly mixing in the other component and then cooling the resulting molten mass until it solidifies. Alternatively, both the film-forming component and the modifying component can be made molten prior to being mixed.

However, we currently prefer to obtain the molecular mixture of the two essential components by forming a solution of the components in a solvent and then evaporating the solution to at least substantial dryness.

In whatever manner the liquid mixture of the film-forming and modifying components is produced, it can then be converted by any suitable technique, e.g. casting, moulding or extrusion, into a solid material of a shape and size to suit the desired end use.

Thus, for example, in accordance with one preferred embodiment, the liquid mixtures are converted into film form by means of a casting process. Preferred compositions in accordance with this invention can be converted into transparent, tough and flexible self-supporting films, of a wide variety of thicknesses, and which have a number of properties which render them advantageous for use in surgical and medical applications.

Thus, for instance, films can be obtained by means of this invention which are non-adherent when dry, thereby facilitating the ease with which they may be handled and applied to the skin surface of a patient, but which when moistened, as by contact with moist tissue, become adherent. Such films may therefore be used as primary wound dressings for superficial wounds such as shallow burns and dermabrasions, without requiring an adhesive coating. The self-adherent properties of the films also allow their use as a secondary dressing or bandage, for example to hold in place a gel-dressing of the kind which is used for the treatment of cavity wounds. The flexibility of the films means that they are readily conformable to wound contours.

Further, the present films are gas and water vapour permeable but preferably have sufficient retained or absorbed water to maintain a high humidity at a wound-dressing interface. At the same time they are sufficiently strong to isolate the wound and provide a barrier to microbial invasion and mechanical stresses. If, as is preferred, the film-forming and film-modifying components are water-soluble, then the films can be readily washed away so that they can be removed without trauma to the patient.

Such properties of the preferred films also make them suitable for use as surgical drapes which are less prone to suffer the disadvantages of conventional drape materials which have been mentioned above.

Another advantage of the preferred films is that a drug can be incorporated in the film at the time of its formation for subsequent release in a controlled manner when the film is placed in contact with moist tissue. Accordingly, drugs such as antibacterials, antiseptics and debriding agents may be incorporated into films to be used as wound dressings, or the films may be used primarily as drug delivery devices for the topical application of a wide variety of drugs, for example as buccal patches for placing in the mouth, as spermicidal contraceptive devices, and as oral and ophthalmic drug delivery devices.

Polymers have been used as matrices for drug delivery systems for many years, for example in micro-particulate form (for administration intravenously), in pre-formed shapes (for administration as pessaries, suppositories or implants), and as oral or transdermal drug delivery devices. Many of these systems have utilised water-insoluble polymers to ensure integrity and controlled drug release and this can be a disadvantage as removal of the material often has to be effected when the drug supply has become exhausted. In the case of implants, this is often impossible or very difficult to achieve. Furthermore, the use of polymeric drug delivery devices in animals has hitherto been relatively limited, primarily because of product cost constraints and difficulties associated with administration of therapy. Such devices need to be made in such a way that the animal host cannot effect removal.

It is possible to combine two or more film compositions to form a laminate. Such laminates may be produced, for example, by casting a film from a first composition and then repeating the process with a second, different film composition, using the first film as a base. This procedure may be repeated as required. Alternatively two or more pre-formed films may be joined together under heat and pressure. An advantage of a laminate is that it allows a desired combination of mechanical and/or physical properties of the individual film compositions to be obtained. For example, a controlled drug delivery device can be provided by using such a laminate, with one or more lamina being selected for slower drug release and one or more lamina being selected for faster drug release.

The materials of the present invention can also be produced in numerous other solid shapes in addition to films and sheets, for instance in order to make implants for drug delivery, contraceptive devices, and products used in surgical intervention to prevent tissue adhesions.

For example, blocks of the material may be produced by successively applying relatively thin liquid layers one upon another until the required block thickness is obtained, each liquid layer being caused to solidify before the next layer is laid down. Such blocks can then be cut to the required dimensions, if need be.

Moulding and extrusion are further techniques which may advantageously be employed to make articles of this invention in a desired shape, form or size.

The first essential component of the present materials is defined as being a compound which has film-forming properties, by which is meant a compound which can form a solution, in a solvent for the compound, which upon evaporation to substantial dryness is capable of forming a self-supporting film.

However, and as will be quite apparent from the preceding discussion, the fact that the first component is defined by reference to its capability of forming films from solution, does not mean either that the present materials must be formed as films, or that a solvent must be used in the preparation of the materials.

It is, of course, necessary that the film-forming compound should be compatible with the selected modifying component, and moreover it is required for medical uses that both components should be non-toxic and non-antigenic.

3

We currently prefer to use the water-soluble compounds hydroxyethylcellulose, hydroxypropylmethylcellulose and/or polyvinyl alcohol as the film-forming ingredient. Low viscosity grade hydroxyethylcellulose with a viscosity (5% w/w aqueous solution at 25°C, Brookfield Model LVF viscometer with No. 4 spindle) in the range 100-180 centipoises (cps), such as that sold by Aqualon (UK) Ltd under the trade mark "Natrosol 250L", is especially advantageous in many instances, particularly where it is desired to form a film from a solution of the film-forming and modifying components, since not only can it be incorporated in relatively high concentrations for good film-forming properties, without unduly increasing the viscosity of the solutions before the drying stage which forms the film, but more particularly because it is highly compatible and miscible with Pluronic F127 which, as will be described below, is our preferred modifier.

Higher viscosity grades of hydroxyethylcellulose may be preferred in other instances, for example to provide a mechanically stronger film or other product or to decrease the rate of drug release from the product. Commercially available examples of such higher viscosity grades of hydroxyethylcellulose are "Natrosol 250G" with a viscosity in the range 260-400 cps for a 2% w/w aqueous solution at 25°C and "Natrosol 250M" with a viscosity in the range 4700-6500 cps for a 2% w/w aqueous solution at 25°C both being sold by Aqualon (UK) Ltd. The above two viscosities, and indeed all the other viscosities in this specification are as determined, at the stated temperature, using a Brookfield Model LVF viscometer with a No. 4 spindle.

In the preferred embodiments of the invention, wherein the molecular mixture is obtained by evaporating a solution of the film-forming and modifying components, the film-forming component preferably should be present dissolved in the solvent in amount of from 2% to 25% by weight. If the amount of the film-former is less than 2%, then the film or other article will generally take too long to dry to self-supporting form to be of commercial value, whilst in any event the product itself may be too weak for any practical purpose. On the other hand, use of more than 25% by weight of the film former is likely to lead to a solution which is so viscous as to be difficult, if not impossible, to form into a film or other required shape. In general, it is preferred that the amount of the film-former in the solution should be from 5% to 15% by weight and advantageously from 8% to 12% by weight in the case of the production of films, for ease and speed of film formation and for providing a film of good strength.

In those cases where the molecular mixture of film-forming and modifying component is obtained from a molten mixture of the components, then the preferred amounts of the film-former will correspond to the preferred amounts stated for the solution embodiment, for essentially the same reasons.

Mixtures of two or more different compounds may, if desired, be used as the film-forming component, in order to obtain specific properties in the final product, e.g. mechanical strength or drug release rate.

As indicated above, the second essential component of the present materials is a block copolymer of polyoxyethylene-polyoxypropylene or a tetrasubstituted derivative of ethylene diamine wherein the substituents are block copolymers of polyoxyethylene-polyoxypropylene. Mixtures of two or more of these compounds may also be used. The compounds act as modifiers which serve to alter the properties of the materials, presumably by influencing the degree of interaction between the component molecules. In particular, in cases where the materials are formed using an aqueous solvent the modifiers are believed to act by influencing the extent of micellar interactions. In any event, it is found that the presence of the defined modifying component is essential in order to obtain a desirable balance of properties. A number of properties are controlled by the presence (and concentration) of the modifier, e.g. strength, extensibility, degree of solubilization of other components such as drugs, and of course the importance or otherwise of such properties will depend on the form and intended use of the final product e.g. extensibility is an important property of films to be used as wound dressings but would be far less important in other cases.

As examples of block copolymers of polyoxyethylene-polyoxypropylene suitable for use as the film-modifying component may be mentioned those available under the trade names "Pluronic" (BASF) and "Synperonic" (ICI).

Examples of suitable tetrasubstituted derivatives of ethylene diamine for use as the film-modifier herein include those available under the trade names "Tetronic" (BASF) and "Synperonic T" (ICI). In general we prefer to use as the film-modifying component water-soluble polyoxyethylene-polyoxypropylene block copolymers with an average molecular weight from 6,000 to 15,000. Examples of such block copolymers are those sold by BASF under the trade names "Pluronic" with the following designations:

| Pluronic Designation | Average MW |
|---|---|
| F68 | 8,400 |
| F77 | 6,600 |
| F88 | 11,400 |
| F98 | 13,000 |
| F108 | 14,600 |
| F127 | 11,500 |

Currently, the most preferred modifier for the manufacture of films is "Pluronic F127". This modifier not only gives improved flowability of liquid mixtures of the film-forming and modifying components, and control of drug solubility and release if a drug is present, but in addition exhibits a very low order of toxicity.

In the preferred embodiments of the invention the modifying component is present in the solution which is evaporated to form the material in an amount from 1% to 40% by weight. If less than 1% by weight of modifier is present then no effective control of properties is achieved. On the other hand, if more than 40% of the modifier is used, then the product is likely to become unworkable. The preferred range for the modifier in solution is 1% to 15% by weight, most preferably from 2.5% to 10% by weight.

Equivalent amounts of the modifying component are preferably employed in the embodiments of the invention wherein the molecular mixture is obtained by cooling a molten mass.

Generally, it can be stated that the most preferred weight ratio of the film-forming component to the modifying component in the molecular mixture is from 1:1.25 to 4.8:1.

In the preferred embodiments of the invention, the solvent used preferably is aqueous and normally will consist of water alone. However, in some instances it may also be advantageous to incorporate a miscible and volatile organic solvent such as ethanol or methylene chloride to improve the rate of evaporation of the solvent during manufacture of the material or to increase the rate of solubilization in the solvent of other components of the composition. It is, however, possible to use an entirely non-aqueous liquid solvent, for instance composed of the above-mentioned organic solvents, although it will then normally be necessary to add moisture to the formed material, as will be described in more detail below.

Other ingredients may optionally be included in the materials of this invention in order to alter their properties. For instance, we have found that incorporating a plasticizer into compositions to form films may enhance the elasticity of the film and the adherence of the film to a wound surface or mucous membrane. Suitable plasticisers include polyethylene glycol, glycerol, sorbitol, ethanolamine and calcium chloride. We currently prefer to incorporate polyethylene glycol (PEG), more especially PEG 400, in amounts ranging from 1% to 10% by weight in those films which are designed to serve as wound dressings, in order to enhance the conformability of the dressing at the wound site.

As already indicated, in some cases it may also be desired to incorporate one or more pharmacologically active compounds in the material. Examples of such active compounds are given later in this specification. Generally, the amount of pharmacologically active compound will be from 0.001% to 35% by weight of the initial liquid mixture.

In order to make the materials of this invention by the currently preferred route, the selected film-forming and modifying components are first dissolved in the solvent, preferably aqueous, in the required amounts. Any optional ingredients are also incorporated into the solvent at this time. Preferably, such optional ingredients also will be soluble in the solvent, but this is not essential. The resulting liquid mixture is then converted into the solid material of this invention by any suitable evaporative technique.

Films in accordance with this invention can be made by applying the liquid mixture as a layer to a surface and then causing or allowing the solvent to evaporate almost completely, to leave a dry film which can be removed from the surface. Such a process of forming films from liquid compositions is known in the art as "solution casting". On the laboratory scale, the liquid mixture can be cast onto any flat, impermeable surface such as a glass plate and the thickness of the applied liquid layer may be controlled by a knife spreader or similar device that allows micrometer adjustments of the blade height above the casting surface. The liquid film may then be allowed to dry in the ambient air or it may be force-dried, e.g. in an oven. For large scale manufacture of the films it is preferred that the liquid composition be cast onto a rotating drum or moving belt so as to permit continuous production. Drying of the film can then be effected by moving the drum or belt through an evaporating hood system, whereafter the film strip can be cut to form wound dressings or drug delivery vehicles, for instance, of a desired shape and size.

The dried films produced by these methods are generally translucent.

Blocks in accordance with this invention can be formed either by evaporating a large volume of the liquid composition in a single stage, or by sequentially evaporating smaller volumes of the solution applied one after the other until a block of desired size has been built up.

Moulding and extrusion are yet further techniques which can be usefully employed to convert a liquid mixture of the film-forming and modifying components into a solid material in accordance with this invention.

It will be appreciated that the materials of the present invention are formed under controlled manufacturing conditions for subsequent use e.g. on human or animal patients. The present materials therefore are to be distinguished from the films which form in situ on a patient when the gel-film wound dressing compositions disclosed in our co-pending European Patent Application No. 90302256.4 (Publication No. 0386960) are used.

We have found that control of the water content in the material is important for its appearance and physical properties, e.g. the tensile strength and elongation of films. For example, over-drying of the material from solution can produce a more opaque and more brittle product, whereas under-drying can produce a clearer, softer and weaker product. We therefore prefer to dry the solution of film-forming and modifying components to form a material with a residual water content of from 2% to 35% by weight, preferably from 2% to 10% by weight and more particularly from 5% to 7.5% by weight. Control of the drying to the desired residual water content is preferably achieved by drying under conditions of controlled humidity.

Alternatively, the material can be dried to a moisture content of less than 2% and then allowed to re-absorb moisture to a desired level, for example by placing the material in a cabinet of controlled humidity. In the case where a non-aqueous solvent is used to form the liquid mixture, or the liquid mixture is formed without using any solvent at all, then such an expedient would normally be followed in order that the material should have a desired moisture level.

When the material is placed on an exuding wound it will absorb moisture from the wound exudate. Accordingly, it is possible to emplace dry or relatively dry material, although it may be somewhat brittle, and in a short while the material will have absorbed moisture from the wound and become more pliable.

The shape and dimensions of the present materials will depend on their intended surgical or medical application. For instance, films which are to serve as primary wound dressings will generally have a thickness in the range from 10 to 500 $\mu$m.

After manufacture, bulk film or blocks in accordance with this invention are cut into strips or other shapes of the required size, and then preferably packed into moisture-impermeable containers for dispensing, in order to maintain the water content of the material at the selected optimum level. For example, flexible sachets of polypropylene foil and polyester laminate are suitably used to package the film products of this invention. Articles of other shapes e.g. formed by moulding or extrusion are also preferably stored in moisture-impermeable containers.

Particularly in the case of films which are to be used as primary wound dressings it is necessary that the dressing should be sterile at the time of administration. We prefer to sterilize the films by gamma irradiation, suitably after they have been packaged.

The packaged films may be stored at room temperature without deleteriously affecting their properties.

As already indicated, the materials of this invention find a variety of uses in human and animal medicine and surgery. Thus, it is possible to provide self-adherent, adhesive- and organic solvent-free primary wound dressings which are easy to apply, even to difficult wound sites, which have the strength and barrier properties which are desired in such dressings, which, being translucent, permit visual observation of the wound healing process, and which can be removed simply by washing away. Similarly, the films are also advantageous for use as secondary wound dressings, for example to hold in place a gel-type wound dressing which has been applied to a deep wound. The present films are particularly adapted for use as secondary dressings in conjunction with the gel- film wound dressings which are described and claimed in our co-pending European Patent Application No. 90302256.4 (Publication No. 0386960) the disclosure of which is hereby incorporated by reference.

Another use to which the present films may be put is as a surgical drape. If desired the films for this purpose may include an antimicrobial agent such as povidone-iodine to protect the patient against infection during an operation.

A wide variety of different pharmacologically active or medically useful compounds may be incorporated into the present materials, either in connection with their use as wound dressings or so as to form drug delivery devices for enteral or parenteral application. Some non-limiting examples are given below:

Topical Delivery to the Skin

Anti-inflammatory drugs such as clobetasone butyrate, cortisone, hydrocortisone, hydrocortisone acetate, betamethasone, betamethasone sodium phosphate, clobetasone butyrate, dexamethasone, prednisone, methyl prednisolone, medrysone, fluorometholone, fluocinolone acetonide, fluocortolone, prednisolone, prednisolone sodium phosphate, prednisolone acetate, triamcinolone, aclometasone dipropionate and the like. NSAI drugs and their salts such as biphenylacetic acid, indomethacin, ketoprofen, ibuprofen, sulindac, piroxicam, fenoprofen, flurbiprofen, naproxen, diclofenac sodium, oxyphenbutazone, diflunisal, fenbufen and the like.

Analgesics such as aspirin, phenylbutazone and salicylates.

Anti-histamines such as mepyramine maleate, antazoline, diphenhydramine hydrochloride, promethazine, dibrompropamidine and the like.

Anaesthetics such as amethocaine, benzocaine, bupivacaine, cocaine, lignocaine, dyclonine hydrochloride, oxybuprocaine, etidocaine, phenacaine hydrochloride, piperocaine hydrochloride, propanocaine hydrochloride, proxymetacaine, hexylcaine hydrochloride, mepivacaine and prilocaine.

Anti-infectives such as iodine, povidone-iodine, benzalkonium chloride, cetyl pyrridinium chloride, chlorhexidine and the like.

Anti-fungals such as benzoyldisulphide, zinc undecylenate, undecylenic acid, tolnaftate, nystatin, natamycin. Imidazoles such as clotrimazole, miconazole, sulconazole nitrate, econazole nitrate, ketoconazole and the like.

Anti-parasitic compounds such as ivermectin.

Anti-viral compounds such as idoxuridine, acyclovir and interferon.

Anti-bacterial compounds such as cefoxitin, imipenem, chlortetracycline, chloramphenicol, neomycin, gramicidin, bacitracin, silver sulphadiazine, gentamicin, framycetin sulphate, kanamycin, tobramycin, nalidixic acid, norfloxacin, fusidic acid and its salts, mupirocin, polymyxin B sulphate, triclosan and the like. Any combination of the above.

Anti-psoriasis compounds such as dithranol.

Anti-acne compounds and keratolytics such as the vitamin A derivatives isotretinoin and tretinoin, resorcinol, minocycline (hydrochloride), erythromycin, doxycycline.

Wound management compounds such as chemotactic agents, desloughing agents, exudate absorbents such as dextranomer and cadexomer iodine and the like, fibrinolytic agents such as streptokinase-streptodornase (Varidase™).

Anti-neoplastic compounds such as methotrexate, adriamycin, 5-fluouracil and the like.

## Buccal and Sublingual Drug Delivery

Anti-anginal drugs such as glyceryltrinitrate, isosorbide dinitrate and the like.

Anti-hypertensives such as nifedipine.

Hormones such as methyl testosterone, testosterone, oxytocin, insulin, estradiol and the like.

Anaesthetics such as benzocaine, lignocaine and the like.

Analgesics such as aspirin, choline salicylate, buprenorphine HCl, methadone HCl and the like.

Nicotine for anti-smoking.

Anti-histamines such as chlorpheniramine, prochlorperazine and the like.

Anti-depressants such as imipramine HCl and the like.

Barbiturates such as amylobarbitone sodium, and the like.

Treatments for mouth ulcers containing combinations of anaesthetics, steroids such as hydrocortisone and the like, and anti-infectives such as minocycline, fusafungine and the like, and antiinflammatories such as carbenoxolone sodium and the like.

Anti-fungals such as miconazole, amphotericin B.

Antibacterials such as chlorhexidine, metronidazole, nimorazole, nystatin, clioquinol and the like.

## For Ophthalmic Drug Delivery

Steroidal anti-inflammatory drugs such as clobetasone butyrate, cortisone, hydrocortisone, hydrocortisone acetate, betamethasone, betamethasone sodium phosphate, dexamethasone, prednisone, methyl prednisolone, medrysone, fluorometholone, fluocortolone, prednisolone, prednisolone sodium phosphate, prednisolone acetate and triamcinolone. Non-steroidal anti-inflammatory drugs and their salts such as biphenylacetic acid, indomethacin, ketoprofen, ibuprofen, sulindac, piroxicam, fenoprofen, flurbiprofen, naproxen, diclofenac and oxyphenbutazone.

Antihistamines and decongestants such as antazoline sulphate, antazoline phosphate and chlorpheniramine.

7

Antibacterial compounds such as beta-lactam antibiotics (carbenicillin, penicillin G, cefoxitin), imipenem and other derivatives of thienamycin, chloramphenicol, tetracyclines such as chlortetracycline hydrochloride and tetracycline hydrochloride, amino-glycoside antibiotics such as gentamicin (sulphate) framycetin (sulphate), neomycin (sulphate), gramicidin, kanamycin, amikacin, sissomicin and tobramycin. Sulphonamides such as sulphacetamide sodium. Colistin and its salts, polymyxin B, vancomycin. Cephalosporin antibiotics such as cephazolin, cephalothin sodium, cephaloridine and the like. Nalidixic acid, norfloxacin and analogues of nalidixic acid.

Miotics and anticholinergics such as bethanechol (chloride), carbachol, demecarium bromide, ecothiapate iodide, methacholine (chloride), di-isopropyl-fluorophosphate, neostigmine (and its salts), physostigmine (and its salts), pilocarpine (and its salts), pyridostigmine bromide. Sympathomimetics such as adrenaline and derivatives, dipivefrine hydrochloride, naphazoline (and its salts), xylometazoline hydrochloride, phenylephrine hydrochloride, tetrahydrozoline hydrochloride and the like.

Mydriatics such as atropine, homatropine, scopolamine, hydroxyamphetamine, cyclopentolate hydrochloride, eucatropine hydrochloride, oxyphenonium bromide, tropicamide and the like.

Other medicaments used in the treatment of eye conditions or diseases such as:

Anti-glaucoma drugs such as carbonic anhydrase inhibitors, for example, acetazolamide, dichlorphenamide, methazolamide and the like. Also, $\beta$-blockers such as timolol, R-timolol and its salts and combinations, e.g. with pilocarpine. Hyperosmotic agents such as glycerol, mannitol and urea.

Anti-allergy drugs such as sodium cromoglycate.

Antifungal agents such as amphotericin B, nystatin, natamycin, miconazole and flucytosine.

Anti-viral compounds such as acyclovir, adenosine arabinoside (Ara-A), idoxuridine, trifluorothymidine, interferon and interferon inducing agents, e.g. Poly I:poly C.

Anti-parasite agents and/or anti-protozoal compounds such as clindamycin and corticosteroid preparations, ivermectin, pyrimethamine and trisulphapyrimidine.

Anaesthetics such as amethocaine, benzocaine, bupivacaine, cocaine, lignocaine, dyclonine hydrochloride, oxybuprocaine, etidocaine, phenacaine hydrochloride, piperocaine hydrochloride, propanocaine hydrochloride, proxymetacaine, hexylcaine hydrochloride, mepivacaine and prilocaine.

Ophthalmic diagnostic agents such as those used to examine the retina, conjunctiva, cornea, lacrimal areas, or to examine abnormal pupillary responses such as fluorescein sodium and rose bengal stains, methacholine, cocaine, adrenaline, atropine and pilocarpine.

Ophthalmic agents used as adjuncts to surgery such as alpha-chymotrypsin and hyaluronidase.

Immunosuppresive agents and anti-metabolites such as azathioprine, methotrexate, cyclophosphamide.

Chelating agents such as EDTA and deferoxamine.

Combinations of the above, e.g. antibiotic/anti-inflammatory.

## For Vaginal Drug Delivery

Anti-infective compounds such as anti-fungal agents nystatin, natamycin, imidazoles such as econazole isoconazole, clotrimazole, miconozole, fluconazole, metronidazole, ketoconazole and the like.

Sulphonamides such as sulphathiazole, sulphacetamide and the like.

Anti-virals such as acyclovir, inosine pranobex, interferon and podophyllotoxin. Antiseptics such as povidone-iodine, chlorhexidine and the like.

Drugs used to treat urinary tract infections such as quinolone compounds such as cinoxacin and nalidixic acid, alkalising agents such as sodium citrate, citric acid, nitrofurans such as nitrofurantoin, hexamine, penicillins such as procaine penicillin, ampicillin and the like, aminoglycoside antibiotics such as gentamicin and the like, cephalosporins and cephamycins, trimethoprim, tetracyclines, polymyxin B sulphate. Urinary analgesics and anti-inflammatory drugs such as aspirin and other NSAI compounds such as mefenamic acid, naproxen sodium and the like.

Anaesthetics such as lignocaine hydrochloride and the like.

Drugs acting on the smooth muscle to induce labour or abortion such as oxytocin, and prostaglandins, such as alprostadil, dinoprost, gemeprost, dinoprostone and the like.

Drugs used to prevent and treat haemorrhage such as ergometrine maleate and/or combined with oxytocin, anti-fibrinolytic drugs such as tranexamic acid, haemostatic drugs such as ethamsylate.

Myometrial relaxants such as ritodrine HCl, beta-agonists such as terbutaline sulphate, isoxsuprine HCl, salbutamol and the like.

Spermicidal contraceptives such as nonoxinols, oxtoxinols and the like.

## For Rectal Drug Delivery

8

Drugs for local therapy or systemic effect can be administered.

Anti-infective compounds such as anti-fungal agents such as metronidazole and the like. Antibiotics such as penicillins, sulphonamides, tetracyclines and the like. Contrast agents such as barium salts and the like.

Anti-inflammatory/analgesics such as aspirin, paracetamol, non-steroidal anti-inflammatory drugs such as indomethacin, naproxen, biphenylacetic acid and the like, narcotic analgesics such as oxymorphone hydrochloride and the like. Corticosteroids such as hydrocortisone, prednisolone, methylprednisolone and the like.

Anaesthetics such as lignocaine and the like.

Anti-asthmatics such as the xanthines such as theophylline, aminophylline and the like.

Anti-convulsant drugs such as diazepam and anxiolytics, anti-psychotic drugs such as chlorpromazine and the like.

Anti-emetics such as cyclizine, domperidone, prochlorperazine and the like.

Anti-histamines such as promethazine and the like.

Barbiturates such as amylobarbitone sodium and the like.

Proteins and peptides such as insulin and the like.

Laxatives such as bisacodyl and the like. Anti-haemorrhoid compounds such as crotamiton and bismuth subgallate.

## For Nasal Drug Administration

Anti-histamines such as antazoline and the like, corticosteroids such as beclomethasone, flunisolide, betamethasone, dexamethasone and the like.

Antibiotics such as mupirocin, neomycin sulphate. Sympathomimetics such as oxymetazoline hydrochloride, tramazoline hydrochloride, phenylephrine hydrochloride, xylometazoline hydrochloride.

Systemic delivery of beta-blockers such as propranolol and the like. Proteins, peptides such as oxytocin and desmopressin, and hormones such as insulin and buserelin acetate. Vaccines.

## For Oral Drug Delivery

Antacids, anti-spasmodics, laxatives, anti-diarrhoeals, anti-anginals, diuretics, anti-hypertensives, anti-coagulants, anti-thrombotics, fibrinolytics, haemostatics, hypolipidaemic agents, hypnotics, anxiolytics, anti-psychotics, anti-depressants, anti-emetics, anti-convulsants, CNS stimulants, analgesics, antipyretics, anti-inflammatories, muscle relaxants, neuromuscular drugs, hormones, hyper- and hypo-glycaemics, thyroid and anti-thyroid drugs, anti-infective compounds, such as anti-biotics, anti-bacterials, anti-fungals, anti-tuberculous and anti-leprotic drugs, anti-malarials, anthelmintics and amoebicides, anti-virals. Vaccines and immunosuppressants. Nutritional compounds such as vitamins, iron, electrolytes, anti-obesity drugs, anabolic drugs. Anti-asthma drugs, expectorants, mucolytics, decongestants and anti-tussives. Anti-allergic drugs. Oral contraceptives. Anti-neoplastic-anti-tumour drugs. Contrast agents such as barium salts and the like.

The rate of release of a drug from the films and other materials of the present invention is dependent not only on the concentration of the drug in the material but also upon such factors as the nature, content and relative concentrations of the film-forming, modifying and optional components, as well as on such factors as article size and shape, e.g. thickness, moisture content, surface area etc. For example, the rate of drug release is usually retarded by an increase in the concentration of film-former, modifier and/or plasticizer, or by an increase in the viscosity of the film-former which is used. Control of factors such as these therefore permits control of the rate of release of a drug to an optimum level to suit the drug concerned and the application to which it is being put.

The invention is illustrated by the Examples which follow:

## Example 1

A film which can be used as a wound dressing or as a surgical drape was prepared as follows:

| Hydroxyethyl cellulose* | 10.0% | 50 g |
| Pluronic F127 | 2.5% | 12.5 g |
| PEG 400 | 4.0% | 20 g |
| Purified water to | 100.0% | 417.5 g |

* "Natrosol 250L"

Hydroxyethyl cellulose (50 g) was slowly and carefully added to the purified water (417.5 g) at room temperature with stirring to produce a clear, colourless solution. PEG 400 (20 g) was added and thoroughly mixed with the hydroxyethyl cellulose solution, and this was transferred to an ice bath and cooled to 3-4°C. Pluronic F127 (12.5 g) was slowly added to the cold solution with gentle mixing, and allowed to hydrate and disperse overnight at 3-4°C to produce a clear, colourless solution.

The resulting film-forming solution was used to prepare films using a "K Hand Coater" (RK Print Coat Instruments Ltd, Royston, Herts, England). This apparatus consists of wire wound "K-bars" and a rubber-faced impression bed. The "K-bars" are of stainless steel, wound with stainless steel wire of selected diameters to give wet films of predetermined thickness. Aliquots of the film-forming solution were poured onto clean glass plates (100 x 200 mm) placed on the impression bed, and taking the appropriate "K-bar" in both hands, the formulation was drawn downwards at a steady speed without rolling it. Excess formulation was removed at the end of its traverse with a slight twist of the K-bar, leaving the bottom edge of the drawdown clear and definite. The glass plates containing wet film were transferred to a convection oven, and the aqueous vehicle allowed to evaporate at 50°C. Drying was complete in 1-2 hours, confirmed by weighing the film on the plates to a constant weight. The thickness of the dry films was 100 $\mu$m.

The films thus obtained were cut into strips 25 mm wide and 200 mm long, and the mechanical properties of the film determined using a J J Lloyd Model M30K Materials Testing Machine interfaced to an IBM personal computer. The specimen was clamped between the jaws of the tester so that the distance between the grips was 100 mm, and the film extended at a constant rate of 300 mm/min. Tensile strength (Newtons) and elongation to break (% of original length) were calculated from the mean of five determinations. Dry film thickness was measured using a micrometer screw gauge.

Typical results obtained for the film composition described above were as follows:

Appearance          : Translucent film
Residual moisture    : 6.5%
Tensile strength     : 15.3 N
Elongation           : 94.8%
Dry film thickness    :100 $\mu$m

The films were soft, but tough, with very good extensibility and adherence properties.

Example 2

A film which can be used as a drug delivery system was prepared and tested according to the methods described in Example 1:

| Hydroxyethyl cellulose* | 10.0% | 50 g |
| Pluronic F127 | 2.5% | 12.5 g |
| Purified water   to | 100.0% | 437.5 g |

* "Natrosol 250L"

Appearance          : Translucent film
Residual moisture    : 6.0%
Tensile strength     : 28.2 N
Elongation           : 12.0%
Dry film thickness    : 100 $\mu$m

The film was hard and slightly brittle.

Example 3

Films were prepared containing the anti-fungal drug metronidazole according to the procedure de-

10

scribed in Example 1 but adding the metronidazole to the water vehicle before the hydroxyethyl cellulose. The resulting films can be used as a wound dressing or as a topical drug delivery system.

Several different films were prepared each containing 0.5% w/w metronidazole, 10% w/w hydroxyethyl cellulose (Natrosol 250L), and the following additional components to demonstrate the effect of formulation composition on the mechanical properties and rate of drug dissolution from the dried films:

| Film Sample No. | % w/w Pluronic F127 | % w/w PEG 400 | Purified Water |
|---|---|---|---|
| 1 | 0 | 4 | to 100% |
| 2 | 2.5 | 4 | to 100% |
| 3 | 5.0 | 4 | to 100% |
| 4 | 2.5 | 0 | to 100% |
| 5 | 2.5 | 2 | to 100% |
| 6 | 2.5 | 6 | to 100% |
| 7 | 0 | 0 | to 100% |

The tensile strength and elongation tests on the resulting films were conducted as described previously. For dissolution testing, 5 g aliquots of the aqueous film-forming composition (ie the composition in solution) were poured into 9 cm diameter plastic petri-dishes kept on a level surface to ensure constant thickness, and the aqueous formulations dried at 50°C for 2 hours in a convection oven.

Dissolution testing was conducted using the dissolution apparatus of design and specifications described in the 1988 British Pharmacopoea (BP), Appendix XIID. The dissolution medium was 900 ml of isotonic phosphate buffer at pH 7.4, prepared from $Na_2HPO_4$ (7.57 g/litre), $NaH_2PO_4.2H_2O$ (2.08 g/litre) and NaCl (4.4 g/litre). Agitation was provided by a BP shaft with paddle attachment positioned 4 cm from the vessel bottom in the dissolution medium, and rotated at 50 rpm. The vessel was maintained at 25°C ± 1°C by means of a thermostatically controlled water bath. This temperature was selected to be similar to skin temperatures.

The petri-dish containing dry film was placed in the bottom of the dissolution vessel with the film side upwards to ensure drug dissolution was from one surface only. Samples of 15 ml volume were drawn off at known time intervals and filtered through a 0.45 $\mu$m Acrodisc™ membrane filter. Metronidazole concentrations were determined spectrophotometrically at 318 nm against a metronidazole standard (0.01 mg/ml) prepared in isotonic phosphate buffer.

The results are shown in Tables 1 and 2 below:

Table 1

| The effect of Pluronic F127 content on metronidazole films | | | |
|---|---|---|---|
| | Sample No. 1 | Sample No. 2 | Sample No. 3 |
| % w/w Pluronic F127 | 0 | 2.5 | 5.0 |
| Appearance of film | Clear, transparent very 'tacky' film | Slightly opaque film | More opaque than Sample No. 2 |
| Residual moisture content | 4-5% | 4-5% | 4-5% |
| Dry film thickness ($\mu$m) | * | 70 | 85 |
| Tensile strength (N) | * | 6.4 | 6.0 |
| Elongation (%) | * | 61.5 | 59.8 |
| Time for 90% of metronidazole to be released (mins) ($t_{90\%}$) | Not tested | 37 | 49 |

* Film was thin and very tacky, so no testing could be performed

The above results show that increasing the Pluronic F127 concentration in the film composition from 2.5% to 5.0% slightly reduced the clarity of the film, but had no significant effect on the mechanical properties of the film (tensile strength and elongation). However, the rate of release (dissolution) of metronidazole from the films was retarded.

Table 2

| The effect of plasticizer content (PEG 400) on metronidazole films | | | | |
|---|---|---|---|---|
| | Sample No. 4 | Sample No. 5 | Sample No. 2 | Sample No. 6 |
| % w/w PEG 400 | 0 | 2.0 | 4.0 | 6.0 |
| Appearance of film | Opaque film | Translucent film | Translucent film | Translucent film |
| Residual moisture content | 4-5% | 4-5% | 4-5% | 4-5% |
| Dry film thickness ($\mu$m) | 60 | 90 | 70 | 75 |
| Tensile strength (N) | 17.6 | 9.4 | 6.4 | 6.2 |
| Elongation (%) | 17.0 | 49.3 | 61.5 | 84.5 |
| Time for 90% of metronidazole to be released (mins) ($t_{90\%}$) | 15 | 18 | 27 | 32 |

The above results show that increasing the PEG 400 content in the film composition, from 0 to 6.0% w/w, produces a film which is weaker but more extensible. Film compositions containing no plasticizer were relatively hard and brittle, whereas film compositions containing 6% plasticizer were soft and weak, but exhibited strong adhesive properties to dry or moist tissue, or to itself. Metronidazole release was retarded with increased PEG 400 concentration.

Sample No. 7, containing no Pluronic F127 and no plasticizer, could not be cast out to produce a satisfactory film due to its poor flow properties and the strong cohesive properties of the material.

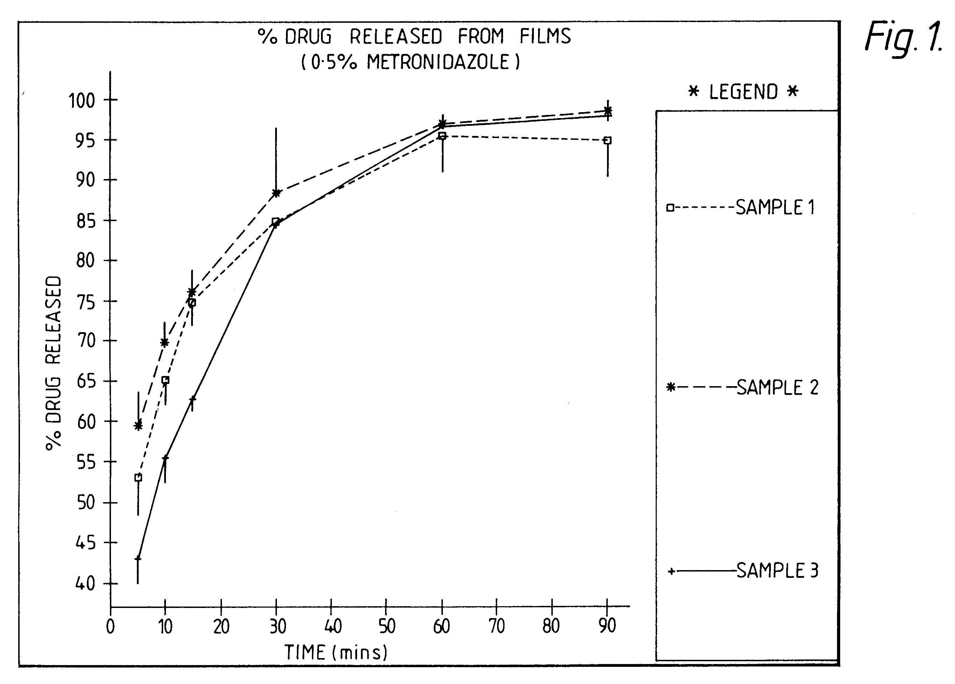

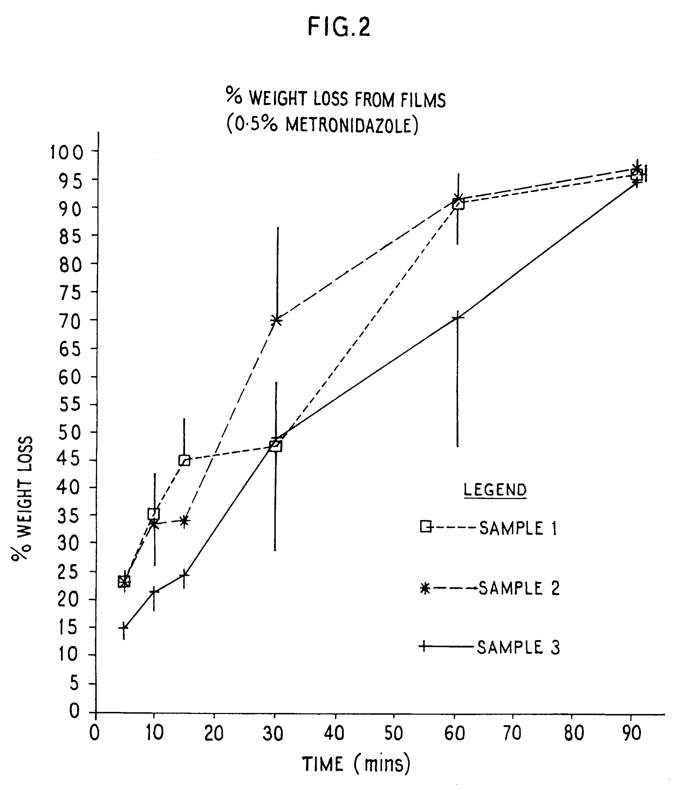

In order to test the mechanism of drug release from these films, dissolution experiments were repeated with each metronidazole film sample to determine the % film dissolved and % drug dissolved. To do this, the experiment was stopped at a known time, and the petri-dish and remaining film recovered from the dissolution vessel. This was dried in a convection oven at 50°C for 2 hours and weighed, and the % film dissolved determined from the difference between this value and the original weight of film used. Figs. 1 and 2 of the accompanying drawings show respectively the % drug released and % weight loss of the films of samples Nos. 1, 2 and 3 above. The determinations were made in triplicate and the graphs show mean ± standard deviation.

Figs. 1 and 2 show similar drug release profiles and similar weight loss profiles for films containing either 0% or 2.5% Pluronic F127. For films containing 5% Pluronic F127, both rate of drug dissolution and rate of weight loss were slower relative to the control.

Example 4

A film composition was prepared containing the anti-inflammatory drug, biphenyl acetic acid (BPAA) for topical delivery to the skin, or as a buccal patch.

| BPAA (as ethanolamine salt) | 0.5% | 1.25 g |
|---|---|---|
| Hydroxyethyl cellulose* | 10.0% | 25.0 g |
| Pluronic F127 | 2.5% | 6.25 g |
| PEG 400 | 4.0% | 10.0 g |
| Purified water   to | 100.0% | 207.5 g |

* "Natrosol 250L"

The film was prepared and tested according to the methods described in Example 3, and possessed the following properties:

Appearance : Translucent film, with a slight yellow tinge
Residual moisture : 4-5% w/w
Tensile strength : 20.2 N
Elongation : 93.5%
Dry film thickness : 250 $\mu$m
Dissolution $t_{90\%}$ : 38 mins

Compared to metronidazole in the same film composition (sample No. 2 of Example 3) the drug release rate is retarded with BPAA, (38 minutes for 90% to be released compared to 26 minutes for metronidazole). The BPAA film is a weaker film, but possesses better extensibility properties.

## Example 5

A film composition was prepared containing a 1:1 mixture of methanol and water as the solvent.

| | | |
|---|---|---|
| Hydroxyethyl cellulose* | 10.0% | 20.0g |
| Pluronic F127 | 2.5% | 5.0g |
| PEG 400 | 4.0% | 8.0g |
| Water | 41.75% | 83.5g |
| Methanol | 41.75% | 83.5g |

*Natrosol 250L

The film was prepared and tested according to the methods described in Example 1. In addition, the residual moisture content of the film was determined using a Mitsubishi Model CA-05 Moisture Meter, by the Karl-Fischer method. The film had the following properties:

Appearance: Translucent, glossy film. Slightly adhesive
Moisture content: 4.9% w/w
Tensile strength: 7.3 N
Elongation: 46.3%
Thickness: 49 $\mu$m

## Example 6

A film composition was prepared containing a 1:1 mixture of ethanol and water as the solvent.

| | | |
|---|---|---|
| Hydroxyethylcellulose* | 10.0% | 5.00g |
| Pluronic F127 | 2.5% | 1.25g |
| PEG 400 | 4.0% | 2.00g |
| Water | 41.75% | 20.88g |
| Ethanol | 41.75% | 20.88g |

*Natrosol 250L

The film was prepared and tested according to the methods described in Example 5, and had the following properties:

Appearance: Translucent, glossy film. Slightly adhesive
Moisture content: 4.8% w/w
Tensile strength: 9.1 N
Elongation: 52.1%
Thickness: 66 $\mu$m

## Example 7

A film composition was prepared, using Pluronic F68 as the film modifying agent.

| | | |
|---|---|---|
| Hydroxyethylcellulose* | 10.0% | 10.0g |
| Pluronic F68 | 2.5% | 2.5g |
| PEG 400 | 4.0% | 4.0g |
| Water | 83.5% | 83.5g |

*Natrosol 250L

The film was prepared and tested according to the methods described in Example 5, and had the following properties:

| | |
|---|---|
| Appearance: | Translucent, slightly adherent film |
| Moisture content: | 8.4% w/w |
| Tensile strength: | 11.5 N |
| Elongation: | 57.6% |
| Thickness: | 67 $\mu$m |

## Example 8

A film composition was prepared, using Pluronic F77 as the film modifying agent.

| Hydroxyethylcellulose* | 10.0% | 10.0g |
|---|---|---|
| Pluronic F77 | 2.5% | 2.5g |
| PEG 400 | 4.0% | 4.0g |
| Water | 83.5% | 83.5g |

*Natrosol 250L

The film was prepared and tested according to the methods stated in Example 5, and had the following properties:

| | |
|---|---|
| Appearance: | Translucent, slightly adherent film |
| Moisture content: | 8.2% w/w |
| Tensile strength: | 10.9 N |
| Elongation: | 60.6% |
| Thickness: | 69 $\mu$m |

## Example 9

A film composition was prepared, using Pluronic F88 as the film modifying agent.

| Hydroxyethylcellulose* | 10.0% | 10.0g |
|---|---|---|
| Pluronic F88 | 2.5% | 2.5g |
| PEG 400 | 4.0% | 4.0g |
| Water | 83.5% | 83.5g |

*Natrosol 250L

The film was prepared and tested according to the methods stated in Example 5, and had the following properties:

| | |
|---|---|
| Appearance: | Translucent, slightly adherent film |
| Moisture content: | 5.0% w/w |
| Tensile strength: | 11.0 N |
| Elongation: | 57.6% |
| Thickness: | 75 $\mu$m |

## Example 10

A film composition was prepared, using Pluronic F98 as the film modifying agent.

| Hydroxyethylcellulose* | 10.0% | 10.0g |
|---|---|---|
| Pluronic F98 | 2.5% | 2.5g |
| PEG 400 | 4.0% | 4.0g |
| Water | 83.5% | 83.5g |

*Natrosol 250L

The film was prepared and tested according to the methods stated in Example 5, and had the following properties:

| | |
|---|---|
| Appearance: | Translucent, slightly adherent film |
| Moisture content: | 4.5% w/w |
| Tensile strength: | 8.9 N |
| Elongation: | 49.0% |
| Thickness: | 69 $\mu$m |

Example 11

A film composition was prepared, using Pluronic F108 as the film modifying agent.

| Hydroxyethylcellulose* | 10.0% | 10.0g |
|---|---|---|
| Pluronic F108 | 2.5% | 2.5g |
| PEG 400 | 4.0% | 4.0g |
| Water | 83.5% | 83.5g |

*Natrosol 250L

The film was prepared and tested according to the methods stated in Example 5, and had the following properties:

| | |
|---|---|
| Appearance: | Translucent, slightly adherent film |
| Moisture content: | 6.0% w/w |
| Tensile strength: | 9.2 N |
| Elongation: | 55.3% |
| Thickness: | 66 $\mu$m |

Example 12

A film composition was prepared, using Natrosol 250G, a higher viscosity grade hydroxyethylcellulose than Natrosol 250L.

| Natrosol 250G* | 5.0% | 5.0g |
|---|---|---|
| Pluronic F127 | 2.5% | 2.5g |
| PEG 400 | 4.0% | 4.0g |
| Water    to | 100.0% | 88.5g |

*The viscosity of Natrosol 250G is 260-400 cps, as a 2% w/w aqueous solution as measured using a Brookfield Model LVF viscometer with No. 4 spindle.

The film was prepared and tested by the methods described in Example 1, and had the following properties:

| | |
|---|---|
| Appearance | : Translucent, slightly adherent film |
| Tensile Strength | : 4.6N |
| Elongation | : 30.2% |
| Dry film thickness | : 48 $\mu$m |

Example 13

A film composition was prepared using a relatively high concentration of Natrosol 250L.

| Natrosol 250L* | 15.0% | 15.0g |
|---|---|---|
| Pluronic F127 | 5.0% | 5.0g |
| PEG 400 | 4.0% | 4.0g |
| Water   to | 100.0% | 78.5g |

*Hydroxyethylcellulose

The film was prepared by and tested by the methods described in Example 1 and had the following properties:

Appearance           : Thick, translucent, tough film
Tensile Strength     : 60.5 N
Elongation           : 6.6%
Dry film thickness   : 425 $\mu$m

Example 14

A composition was prepared with a relatively high concentration of Pluronic F127.

| Natrosol 250L* | 2.0% | 2.0g |
|---|---|---|
| Pluronic F127 | 30.0% | 30.0g |
| PEG | 4.0% | 4.0g |
| Water   to | 100.0% | 64.0g |

*Hydroxyethylcellulose

The film was prepared by the method described in Example 1.

Example 15

A film was prepared from two compositions, containing different grades of hydroxyethylcellulose (Natrosol), to demonstrate the principle of lamination.

| Composition (a) | | |
|---|---|---|
| Natrosol 250L | 10.0% | 10.0g |
| Pluronic F127 | 2.5% | 2.5g |
| PEG 400 | 4.0% | 4.0g |
| Water   to | 100.0% | 83.5g |

| Composition (b) | | |
|---|---|---|
| Natrosol 250G | 5.0% | 5.0g |
| Pluronic F127 | 2.5% | 2.5g |
| PEG 400 | 4.0% | 4.0g |
| Water   to | 100.0% | 88.5g |

Composition 15(a) was spread to form a film, then dried, as described in Example 1. The second composition was prepared and dried in a similar way and then firmly laid over the first film, which had been slightly moistened with water.

The resulting laminated film had the following properties:

Appearance           : Translucent, slightly adherent film
Tensile strength     : 13.5 N
Moisture content     : 9.31%
Dry film thickness   : 138 $\mu$m

Such a laminate would be useful as a carrier for one or more drugs when it was desired to provide

17

different rates of release of the drug or drugs.

Example 16

A moulded article was prepared from the same composition as described in Example 13.

The aqueous solution containing the Natrosol 250L, Pluronic F127 and PEG 400 was poured into a cylindrical mould having an internal diameter of 23 mm and a height of 15 mm. The mould was then placed in an oven at 50°C until the composition had dried.

The resulting cylindrical block removed from the mould was tough, clear and flexible but had shrunk to approximately one third of the height of the mould due to water loss.

The following two examples illustrate the preparation of an article in accordance with this invention from starting compositions containing no or only a small amount of solvent, by forming a melt of the modifying component of the composition.

Example 17

An article was prepared from the following ingredients:

| Pluronic F127 | 75.5% | 20.0g |
|---|---|---|
| PEG 400 | 15.1% | 4.0g |
| Hydroxyethylcellulose* | 9.4% | 2.5g |

*Natrosol 250L Pharm.

The Pluronic F127 was melted in a water bath at 70°C. The PEG 400 was then slowly stirred into the molten mass, followed by the hydroxyethylcellulose. The resulting mixture was maintained at 70°C for 15 minutes to allow the hydroxyethylcellulose to disperse homogeneously, and then the hot mixture was quickly poured onto a Petri dish which had been cooled on a bed of ice. An opaque, white solid with a brittle, waxy appearance formed as a result of this rapid cooling of the molten mixture.

The viscosity of the molten mass formed at 70°C was such that the composition could be extruded to form rods or other desired shapes in larger scale manufacturing operations.

Example 18

An article was prepared from the following ingredients:

| Pluronic F127 | 42.55% | 10.0g |
|---|---|---|
| PEG 400 | 42.55% | 10.0g |
| Hydroxyethylcellulose* | 10.60% | 1.0g |
| Water | 4.3% | 2.5g |

*Natrosol 250L Pharm.

The method described in Example 17 was followed, except that the water was pre-mixed with the PEG 400 and added to the molten Pluronic F127.

The solid product which was obtained upon cooling was similar to that obtained from the solventless composition employed in Example 17.

**Claims**

1. A solid material comprising a molecular mixture of (a) one or more compounds having film-forming properties, with (b) one or more modifying compounds selected from (i) block copolymers of polyoxyethylene-polyoxypropylene and (ii) tetrasubstituted derivatives of ethylene diamine wherein the substituents are block copolymers of polyoxyethylene-polyoxypropylene, the weight ratio of said component (a) to said component (b) in said mixture being from 1:20 to 25:1.

2. A material according to Claim 1, obtained by forming a liquid mixture of said components (a) and (b)

18

and then causing said mixture to solidify.

3. A material according to Claim 2, obtained by evaporating to at least substantial dryness a solution of said components (a) and (b) in a solvent.

4. A material according to Claim 3, wherein said solution comprises water in which is dissolved (a) from 2% to 25% by weight of one or more water-soluble compounds having film-forming properties and (b) from 1% to 40% by weight of one or more water-soluble modifying compounds selected from (i) block copolymers of polyoxyethylene-polyoxypropylene and (ii) tetrasubstituted derivatives of ethylene diamine wherein the substituents are block copolymers of polyoxyethylene-polyoxypropylene.

5. A material according to Claim 4, wherein said aqueous composition comprises from 5% to 15% by weight of said component (a).

6. A material according to Claim 5, wherein said aqueous composition comprises from 8% to 12% by weight of said component (a).

7. A material according to one of Claims 4-6, wherein said aqueous composition comprises from 1% to 15% by weight of said modifying component (b).

8. A material according to Claim 7, wherein said aqueous composition comprises from 2.5% to 10% by weight of said modifying component (b).

9. A material according to Claim 2, obtained by cooling a liquid composition comprising at least one of components (a) and (b) in molten form at an elevated temperature.

10. A material according to Claim 9, obtained by cooling a liquid composition comprising molten modifying component (b) into which has been mixed said film-forming component (a).

11. A material according to any preceding claim and having a moisture content of from 2% to 35% by weight, preferably from 2% to 10% by weight, more particularly from 5% to 7.5% by weight.

12. A material according to Claim 11 when appendant to any one of Claims 3-8, wherein said moisture content is the residual moisture content of said material following the evaporation of said solvent.

13. A material according to any preceding claim, wherein said component (a) comprises one or more compounds selected from hydroxyethylcellulose, hydroxypropylmethylcellulose and polyvinyl alcohol.

14. A material according to Claim 13, wherein said component (a) is a low viscosity grade hydroxyethylcellulose.

15. A material according to any preceding claim, wherein said modifying component (b) comprises a polyoxyethylene-polyoxypropylene block copolymer having an average molecular weight of about 11,500.

16. A material according to any preceding claim, comprising also a plasticizer, preferably polyethylene glycol.

17. A material according to any preceding claim, comprising also at least one compound having pharmacological activity or medical use.

18. A material according to any preceding claim, in the form of a shaped article.

19. A material according to Claim 18 in the form of a film or sheet.

20. A material according to Claim 19 in the form of a moulded or extruded article.

21. A material according to Claim 20 in the form of a cast block.

19

**22.** A material according to Claim 19, which comprises a laminate of two or more of said films or sheets.

**23.** A wound dressing, comprising a sterilized film or sheet material according to Claim 19.

**24.** A wound dressing according to Claim 23, which has been cut to a desired size and shape.

**25.** A drug delivery vehicle, comprising a sterilized material according to Claim 17.

**26.** A drug delivery vehicle according to Claim 25, which has been cut to a desired size and shape.

**27.** A material according to Claim 1 and substantially as described in any one of the Examples herein.

Fig. 1.

% DRUG RELEASED FROM FILMS
( 0·5% METRONIDAZOLE )

* LEGEND *

□-------SAMPLE 1

*-------SAMPLE 2

+-------SAMPLE 3

EP 0 481 600 A2

# FIG.2

% WEIGHT LOSS FROM FILMS
(0·5% METRONIDAZOLE)

LEGEND

□-----SAMPLE 1

*---*SAMPLE 2

+-----SAMPLE 3

% WEIGHT LOSS

TIME (mins)